# EUROPEAN PATENT APPLICATION

(11) **EP 3 466 920 A1**
(43) Date of publication of application: **10.04.2019**
(21) Application number: 17805923.4
(22) Date of filing: 18.05.2017
(51) Int. Cl.: C07C 213/08, C07C 215/68, B01J 23/44

(54) **METHOD FOR OBTAINING -AMINO ALCOHOLS**

(30) Priority: 31.05.2016 ES 201600468
(71) Applicant: Universitat de València, 46010 Valencia (ES)
(72) Inventor: BALLESTEROS CAMPOS, Rafael, 46100 Burjassot (ES); ABARCA GONZÁLEZ, Mª Belén, 46100 Burjassot (ES); BALLESTEROS GARRIDO, Rafael, 46100 Burjassot (ES); LLABRÉS CAMPANER, Pedro Juan, 46100 Burjassot (ES)
(74) Representative: Cueto, Sénida
(86) International application number: PCT/ES2017/070333
(87) International publication number: WO 2017/207839

(57) **Abstract**

The invention relates to a method for obtaining β-amino alcohols, in which the following steps are performed using amines and a diol/water mixture: adding the diol/water mixture; subsequently, carrying out the reaction at a temperature between 130 and 200°C, for between 2 and 24 hours; filtering the reaction product, comprising β-amino alcohol, using at least one first hexane extraction method, followed by at least one second ethyl acetate extraction method, in order to purify the resulting β-amino alcohol.

## Description

### Field of the invention

The present invention relates to a method for obtaining β-amino alcohols. The invention is included within the sector of the chemical-organic industry, specifically, the area related to the manufacture of biologically important compounds such as amino acids or morpholines.

### Background of the Invention

A technique widely used in organic synthesis of products of industrial interest is the activation of alcohols for amine alkylation by hydrogen self-transfer processes or reactions (Scheme 1). These processes are also known as hydrogen lending or self-supply system of active hydrogens. Samples of this are the works of J. Williams *et al* (1); Krische *et al* (2) and M. Yus *et al* (3). One of the advantages, among others, of these processes is that the use of more toxic alkyl halides is avoided. Within the type of alcohols used as starting material for this type of reactions, easily oxidizable alcohols, for example, benzyl alcohol and derivatives, comprised most of the results described. The use of alkyl alcohols represents a greater challenge.

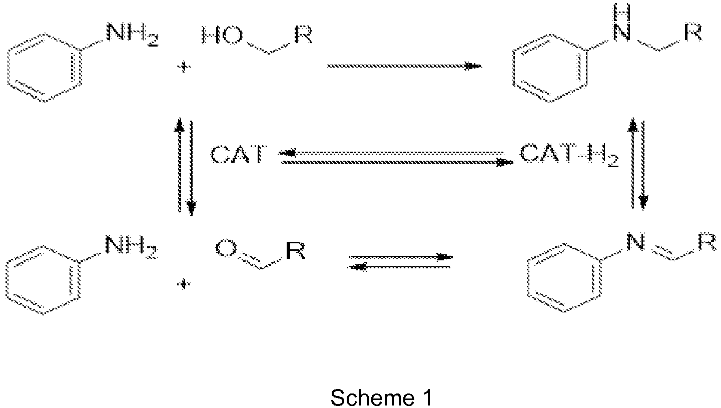

These processes or reactions are performed through the use of catalysts. In the state of the art, catalysts with a greater dehydrogenation potential are described. Examples of those are described by Q. Yang *et al* (4). However, most of the catalysts, the use of which has been published are homogeneous catalysts, such as those described by Yang *et al.* (4). These catalysts consist of a complex of a transition metal with nitrogenated ligands, or phosphorus. Homogeneous catalysts, although they are very effective, have a limited industrial applicability due to their cost (most are complexes of Os, Ru, Ir), their instability, and the impossibility of reusing them. In addition, many of these hydrogen self-transfer reactions require the participation of co-reagents such as alkali metals tert-butoxide and organic solvents.

Due to the interest of this type of reactions (Scheme 1), different heterogeneous catalyst systems have also been used, although basically starting from benzyl alcohols, for example, K. Shimizu (5). The heterogeneous systems are based on transition metals (gold, iridium, palladium, ruthenium ...) supported on alumina, titania or ferrite.

In general, the use of diols as a starting material, specifically ethylene glycol, in this type of reactions (Scheme 1) can lead to the production of β-amino alcohols in one step. However, it represents a challenge or obstacle for two reasons. On the one hand, ethylene glycol is much more difficult to activate than benzylic alcohols. The products of the reaction, the β-amino alcohol or the 2-hydroxyethanal (Scheme 2), have an alcohol group that can be activated by the catalyst, what means that they could be degraded. On the other hand, and being a technical disadvantage to be taken into account, by using very potent catalysts, ethylene glycol can also be degraded.

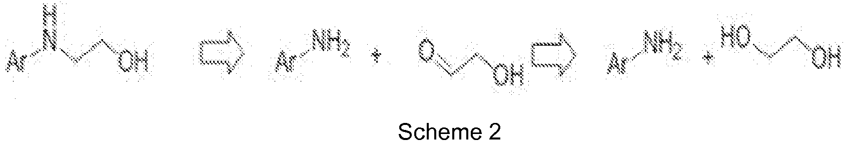

β-amino alcohols represent a product of highly industrial interest given its applicability in the synthesis of biologically relevant compounds such as amino acids or morpholines. For this reason, it is necessary and has a special relevance the supply of a method that allows obtaining β-amino alcohols in the least expensive way, and as respectful as possible with the environment.

β-amino alcohols have potential in the preparation of heterocyclic carbenes, which are those used in many catalysis reactions. An example of their many applications can be found in the work of T. J. Donohoe *et al* (6). However, in this publication there is no reaction that meets the criteria of green chemistry. Green chemistry is known in the art as ecological chemistry, that which respects the environment, and, far less, that uses ethylene glycol as starting material in the preparation of β-amino alcohols.

Two references, as examples of activation reaction of ethylene glycol, for obtaining another type of product have been found: first, the publication of S. Michlik *et al* (7), which, to synthesize pyrrole, uses a homogeneous catalyst (an iridium complex) that is very expensive, unstable and with few possibilities of use on an industrial scale, and where the product is generated by 32%; and a second, patent document CN103539718, from China Petroleum & Ch. Sinopec Shanghai Research Institute, where they assume that the β-amino alcohol is a by-product of a catalysis at 300°C, the objective of which is the synthesis of indole and use of a metal (Ag, Cu) as a catalyst. However, β-amino alcohol is not isolated in this last reference.

The introduction of an extra stage with diethyl carbonate to activate ethylene glycol has been described as an alternative for industrial application. According to ML Kantam *et al* (8) and AB Shivarkar *et al* (9), ethylene glycol is transformed into 1,3-dioxolan-2-one. However, this method reduces atomic efficiency and, furthermore, is far from providing acceptable yields.

It is important to bear in mind that, except for the examples cited above (Michlik and the Chinese patent document), which use ethylene glycol, no real method is known that obtains β-amino alcohols directly from ethylene glycol as starting material without any modification, in a heterogeneous catalytic system.

While there are many reactions published, to activate any type of alcohol by different routes, all of them give rise to compounds other than β-amino alcohols. An example is the publication of A. Corma *et al* (10), wherein Pd/MgO is used to react ethylene glycol with amines; However, it is not possible to obtain amino alcohols, but piperazines or other derivatives as the result of the ethylene glycol polyfunctionalization. In addition, a fundamental problem of said reaction is that ethylene glycol can be degraded, giving rise to polymers or gas mixtures (CO/CO₂), depending on the catalyst potential. Although there are many and varied references on the processes of hydrogen self-transfer to give amines, regarding β-amino alcohols there are only the two examples cited above.

Therefore, a method capable of monofunctionalizing ethylene glycol and in which the product obtained is not degraded, in this case, a β-amino alcohol, is required.

Protocols for the generation of amines by green methodologies, that is, methodologies that respect the environment have been developed. For example, two of the latest publications, cited below, represent the first developments in the field of hydrogen self-transfer: "An efficient one pot transfer hydrogenation and N-alkylation of quinolines with alcohols mediated by Pd/C/Zn" by B. Abarca, R. Adam, R. Ballesteros, Organic Biomolecular Chemistry, 2012, 10, 1826-1833. DOI: 10.1039/C1OB05888F and "Triazolopyridines. Part 30.1. Hydrogen transfer reactions; pyridylcarbene formation". B. Abarca; R. Adam; S. Alom; R. Ballesteros; S. Lopez-Molina. ARKIVOC, Vol. 2014, Issue 2, pp. 175-186.

However, in none of these two publications β-amino alcohols were obtained. It is true that the Pd/C catalyst is capable of performing hydrogenation-dehydrogenation processes. Still, in the case of these two publications, tertiary amines or pyridines were the products obtained. In the particular case of the first of the two publications cited above, a combination described as Pd/C/Zn is used. However, it must be taken into account that this combination is not a catalyst, since Zn (metal in oxidation state 0) contributes electrons that are used to reduce quinolines (becoming Zn⁺²), being a reagent (in addition, used in a large excess) and cannot be recovered at the end of the reaction, also generating scaling problems due to metals instability in oxidation state 0 at high temperatures. In this first publication, ethylene glycol is used, but the preparation method does not use, as starting materials, the amines, but the tetrahydroquinolines, which have different properties and result in a product different from the β-amino alcohols. Keep in mind that, tetrahydroquinolines are compounds much more difficult to degrade and have a higher resistance to generate polyfunctionalized systems. Therefore, although the above mentioned methodologies are relatively innovative, taking into account the large number of catalysts for amines functionalization, these methodologies do not allow to obtaining β-amino alcohols in any case.

Thus, it has not been found in the state of the art a publication, teaching or patent document that manages to functionalize ethylene glycol to generate β-amino alcohols, through the use of heterogeneous catalytic systems.

Currently, tendency is directed to the production of catalysts with greater potential. This represents a problem when carrying out reactions with more than one active center, such as β-amino alcohols formation from ethylene glycol. This is due to the fact that very active or high potential catalysts degrade the products obtained, inducing multifunctionalizations, as is the case, for example, of the publication by Corma *et al* (10).

The present invention solves the aforementioned problems and satisfies the need for a method to obtain β-amino alcohols which is ecological, carried out in a heterogeneous catalyst system, at lowest economic cost possible, and wherein the obtained product is not degraded.

### DESCRIPTION OF THE INVENTION

The present invention provides a method for obtaining β-amino alcohols, which, starting from an amine and a diol/water mixture, is carried out in a heterogeneous catalyst system and comprises the following steps:
- a solid catalytic mixture comprising a supported metal and a solid inorganic coactivator with an acid-base character is added to the starting amine,
- then the diol/water mixture is added;
- subsequently, the reaction is carried out, at a temperature between 130 and 200°C, for 2 to 24 hours.
- the reaction product, based on β-amino alcohol, is filtered through a microporous filter, by at least one extraction with hexane and, subsequently, at least a second extraction with ethyl acetate, in order to purify the 3 -amino alcohol obtained.

Therefore, the present method poses a turning point in the design of catalysts used in processes of hydrogen self-transfer. On the other hand, it is the only example of using a heterogeneous catalyst system that is capable of mono functionalizing ethylene glycol to generate β-amino alcohols without degrading the resulting product (β-amino alcohol).

By means of this method, a catalytic mixture comprising a metal, supported on a substrate, and an inorganic solid coactivator, the function of which is also catalytic, is used. Optionally, the metal is selected from the metals of groups IIIB to IIB of the periodic element system. Also, optionally, this metal may be an oxide or a hydroxide of said transition metals, or a Pt-Sn alloy. Preferably, the supported metal is a metal selected from the following elements: Pd, Au, Pt, Cu, Ag, Ru, oxides and hydroxides thereof.

Optionally, the metal is supported on a solid substrate that is selected from the following elements: TiO₂, Al₂O₃, SiO₂, Fe₃O₄, Fe₂O₃, MgO, Ga₂O, CeO₂, NiO, zeolites, graphite, graphene, graphene oxide, diamond, carbon nanotubes, carbon black.

This catalytic mixture, that is, the metal supported on a solid substrate and the solid inorganic coactivator, enables the diol present in a diol/water mixture to be activated, thus generating aldehydes and hydrogen. In the presence of aromatic amines, imines are formed, which are hydrogenated (with the same hydrogen that had been generated in the first part) to obtain a secondary amine and a water molecule as a by-product. This procedure makes it possible to obtain β-amino alcohols, avoiding polyfunctionalization, since by passing through the imine, the reaction is more difficult for the product. Furthermore, by using diol as a solvent, product degradation by the catalyst itself is prevented.

Optionally, after obtaining the β-amino alcohols, the catalytic mixture (metal supported and solid inorganic coactivator with acid-base character) is recovered through a filtration and washing operation with hexane/ethyl acetate and, subsequently, drying at a temperature between 140 and 160°C, for 2 to 6 hours.

Optionally, the inorganic solid coactivator with acid-base character is selected from the following oxides: CaO, BaO, ZnO, CeO₂, Al₂O₃, MgO, TiO₂, SiO₂. Preferably, ZnO, SiO₂ and Al₂O₃ are selected. These solid coactivators, being in their most stable oxidation state and given the reaction conditions, remain unchanged throughout the catalytic method and act (despite their quantity) as catalysts. Therefore, they are denoted as coactivators. All solids that cannot withstand these conditions (metals in low oxidation states or in metallic form) are excluded. ZnO is selected as the preferred coactivator option.

Optionally, the catalytic mixture that is added to the amine comprises palladium supported on carbon and ZnO (inorganic solid coactivator).

By using this catalytic mixture (supported catalyst and inorganic solid coactivator with acid-base character), β-amino alcohols are obtained because the coactivator helps the supported metal to carry out the first stage. However, the coactivator is composed by two independent solids, therefore, the reaction it is much softer and the products are not degraded (β-amino alcohols). This mixture of two solids is extremely rare in catalysis, in practically 100% of the catalysts only one of the compounds used is solid. In the present invention there are two solids. This, logically, leads to longer reaction times, remaining in any case at acceptable values (12-70 h).

It has been discovered that the reaction where a catalyst is used and a coactivator of the ethylene glycol participates, playing a catalytic function, that is to say, without being transformed throughout the reaction, surprisingly allows to obtain β-amino alcohols.

And, in addition, this coactivator is not able to degrade these products.

Therefore, amino alcohols are obtained and isolated by a catalytic mixture in a heterogeneous system, contrary to what happens in practically all the reactions with ethylene glycol described so far in the prior art.

Optionally, the diol comprised in the diol/water mixture may be one from the following group consisting of: 1,2-ethanediol (ethylene glycol), 1,2-propanediol, 2,3-butanediol, 1-phenylethane-1,2-diol, 1,3-propanol, 1,2-cyclohexanediol, 1,3-butanediol and glycerol triol, as well as cyclic derivatives which, by transformation, produce diols, such as styrene oxide. Preferably, 1,2-ethanediol (ethylene glycol) and 1,2-propanediol are selected. The mixtures comprise different proportions, from 1/100 (diol/water) to 100/1, v/v. In no case are other solvents used, far less halogenated solvents.

Difficult to oxidize amines include amines that cannot be rapidly converted into an imine through de-hydrogenation. Optionally, as the starting material of the present invention, aromatic amines of the formula H₂N-Ar are selected, where Ar (aryl group) can be phenyl, ortho/meta/para methylphenyl, ortho/meta/para nitrophenyl ortho/meta/para fluorophenyl, ortho/meta/for aminophenyl, ortho/meta/para trifluoromethylphenyl, ortho/meta/para trifluoromethoxyphenyl, 2,3; 3,4; 4,5; 2.4; 2,5 dimethylphenyl, 3,4,5-trimethylphenyl, anthracyl or naphthyl. Optionally, tertbutylamine can be selected.

According to another optional embodiment, the amine is a heterocyclic amine. For example, pyridines, thiophenes, thiazoles, triazoles, or quinolines. Optionally, tetrahydroquinoline can be selected.

The method is characterized by being a catalytic reaction in a heterogeneous system, that is, both the catalyst itself and the coactivator can be recovered by filtration and be reused. In addition, diol pre-activation is not required. Both the reagents and the solvents do not have halogen atoms, and working temperatures are industrially available. In addition, yields are superior to all those published to date for said reaction or derived ones, with diethyl carbonate. It is important to note that the mixture to which this invention refers, is the only one capable of activating diols in a controlled manner avoiding polymerizations.

Advantages of the present procedure with respect to the state of the art:
- Cost reduction: water and ethylene glycol are economically acceptable; The present method for obtaining β-amino alcohols is the only heterogeneous protocol that does not require activation with diethyl carbonate at high temperatures. No transition metal in solution is used, what significantly reduces the cost of the process. The reaction does not require phosphorus ligands to work, what also has an impact on the total method cost. No molecule used in this method requires anhydrous conditions or an inert atmosphere. Both the catalysts and the reagents, the solvents and the products are stable to air and moisture, and can be stored in regular containers. That is, the use of a glove chamber is not required to preserve the catalysts, as it is the case with many transition metal complexes that are used in homogeneous catalysis. Therefore, the indirect costs of applying the methodology are greatly reduced. The products used are commercially available and used without any extra purification. No pre-treatment of any of the components of this application is necessary.
- Waste reduction: because this method is characterized by being a heterogeneous catalysis, the catalyst can be removed without much difficulty; it can be reactivated and reused, thus minimizing waste and maximizing its price. On the other hand, the present invention allows the solvent not to be excessively degraded, by generating clean raw reaction products and very few by-products. In addition, it is possible to purify the products by selective extraction, a first one, to remove impurities and a second one, which extracts the amino alcohol.
- Halogenated compounds reduction: no haloderivative compound (carcinogenic) is used, neither as a reagent nor as a solvent in the extraction or purification. The catalyst can be reactivated with ethyl acetate.
- Atomic economy: the diol acts as solvent and reagent, being also activated without chemical transformation; In addition, the reaction only generates as a by-product a water molecule.
- Moderate temperatures: the present invention allows a method of use, wherein the working temperature range is comprised between 130 and 200°C, preferably a working temperature around 150°C, and can be easily applied at industrial level.

### Exemplary embodiments

In a Teflon vessel the starting amine (1 mmol), a palladium on carbon catalyst, Pd/C (7%), and ZnO (3 eq) were mixed, and, secondly, 12 ml of a 50% v/v ethylene glycol/water mixture was added to form a reaction mixture. The reaction mixture was placed in an autoclave, which was closed and placed in an oven at 150°C, for 24 h. It was allowed to cool to room temperature, for about 2 h.

The reaction mixture was subjected to filtration through a microporous 0.45 µm polytetrafluoroethylene syringe filter, PTFE, (VWR International). The reaction mixture was then extracted with ethyl acetate, EtOAc, (3x30 ml). The organic phase was dried with Na₂SO₄, gravity filtered, and the solvent was evaporated in vacuum.

The raw reaction product was purified in a chromatotron, using hexane and EtOAc as eluents, obtaining pure amino alcohols.

### 2-p-tolylaminoethanol, starting from p-tolylamine and ethylene glycol

¹H NMR (300 MHz, CDCl₃) δ: 7.01 (d, 2H, J = 8 Hz, ArH), 6.61 (d, 2H, J = 8 Hz, ArH), 3.80 (t, 2H, J = 5 Hz, CH₂), 3.27 (t, 2H, J = 5 Hz), 2.9 (br s (broad singlet), 2H, NH and OH), 2.26 (s, 3H, Me)
¹³C NMR (75 MHz, CDCl₃) δ: 146.0(1C, C), 130.0(1C, C), 127.4(1C, CH), 113.7(1C, CH), 61.4(1C, CH₂), 46.7(1C, CH₂), 20.6(1C, CH₃),
Isolated yield: 84%
Isolated yield at 1 gram scale, with selective extraction, two extractions with hexane followed by two extractions with ethyl acetate (these last two ones contain the product) 45%.

### 2-o-tolylaminoethanol, starting from o-tolylamine and ethylene glycol

¹H NMR (300 MHz, CDCl₃) δ: 7.03 (m, 2H), 6.60 (m, 2H), 3.78 (t, J=5.1 Hz, 2H), 3.26 (t, J=5.1 Hz, 2H) 2.09 (s, 3H).

¹³C NMR (75 MHz, CDCl₃) δ: 145.9 (1C, C), 130.2 (1C, C), 127.1 (1C, CH), 122.6 (1C, CH), 117.6 (1 C, CH), 110.2 (1C, CH), 61.1 (1C, CH₂), 46.1 (1C, CH₂), 17.7 (1C, CH₃).

HRMS [M+H+]: 152.0994
Isolated yield: 28%

### 2-m-tolylaminoethanol, starting from m-tolylamine and ethylene glycol

¹H NMR (300 MHz, CDCl₃) δ: 7.00 (dd, J=11.0;5.1 Hz, 1H), 6.44 (m, 3H), 3.71 (t, J=5.2 Hz, 2H), 3.19 (t, J=5.2 Hz, 2H), 2.82 (s, 1H), 2.20 (s, 3H).

¹³C NMR (75 MHz, CDCl₃) δ: 148.44 (1C, C), 139.54 (1C, C), 129.59 (1C, CH), 119.41 (1C, CH), 114.54 (1C, CH), 110.90 (1C, CH), 61.71 (1C, CH₂), 46.66 (1C, CH₂), 22.00 (1C, CH₃).

Isolated yield: 39%
2-((2,3-dimethylphenyl)amino)ethanol, starting from 2,3-dimethylaniline and ethylene glycol
¹H NMR (300 MHz, CDCl₃) δ: 7.04 (t, J = 7.8 Hz, 1H), 6.64 (d, J = 7.5 Hz, 1H), 6.56 (d, J = 8.1 Hz, 1H), 3.87 (t, J = 5.2, 2H), 3.34 (t, J = 5.2, 2H), 2.30 (s, 3H), 2.09 (s, 3H). ¹³C NMR (75 MHz, CDCl₃) δ: 146.06 (1C, C), 136.87 (1C, C), 126.29 (1C, CH), 121.15 (1C, C), 120.03 (1C, CH), 108.55 (1C, CH), 61.37 (1C, CH₂), 46.48 (1C, CH₂), 20.81 (1C, CH₃), 12.65 (1C, CH₃).

HRMS [M+H+]: 166.1219
Isolated yield: 35%

### 2-((2,4-dimethylphenyl)amino)ethanol, starting from 2,4-dimethylaniline and ethylene glycol

¹H NMR (300 MHz, CDCl₃) δ: 6.94 (d, J = 8.1 Hz, 1H), 6.91 (s, 1H), 6.58 (d, J = 8.0 Hz, 1H), 3.86 (t, J = 5.2, 2H), 3.33(t, J = 5.2, 2H), 2.24 (s, 3H), 2.15 (s, 3H).

¹³C NMR (75 MHz, CDCl₃) δ: 143.77 (1C, C), 131.27 (1C, CH), 127.47 (1C, CH), 126.97 (1C, C), 122.99 (1C, C), 110.61 (1C, CH), 61.37 (1C, CH₂), 46.53 (1C, CH₂), 20.46 (1C, CH₃), 17.58 (1C, CH₃).

HRMS [M+H+]: 166.1217
Isolated yield: 23%

### 2-((3,5-dimethylphenyl)amino)ethanol, starting from 3,5-dimethylaniline and ethylene glycol

¹H NMR (300 MHz, CDCl₃) δ: 6.43 (s, 1H), 6.31 (s, 2H), 3.80 (t, J=5.2 Hz, 2H), 3.28 (t, J=5.2 Hz, 2H), 2.27 (s, 6H).

¹³C NMR (300 MHz, CDCl₃) δ: 148.28 (1C, C), 139.06 (2C, C), 120.08 (1C, CH), 111.37 (2C, CH), 61.35 (1C, CH₂), 46.31 (1C, CH₂), 21.56 (2C, CH₃).

HRMS [M+H+]: 166.1219
Isolated yield: 30%

### 2-((3,4-dimethylphenyl) amino) ethanol, starting from 3,4-dimethylaniline and ethylene glycol

¹H NMR (300 MHz, CDCl₃) δ: 6.96 (d, J=8.0 Hz, 1H), 6.50 (d, J=2.4 Hz, 1H), 6.44 (dd, J=8.0;2.5, 1H), 3.81 (t, J=5.4 Hz, 2H), 3.28 (t, J=5.3 Hz, 2H), 2.72 (s, 1H), 2.21 (s, 3H), 2.17 (s, 3H).

¹³C NMR (300 MHz, CDCl₃) δ: 146.33 (1C, C), 137.52 (1C, C), 130.45 (1C, CH), 126.22 (1C, C), 115.40 (1C, CH), 110.96 (1C, CH), 61.43 (1C, CH₂), 46.69 (1C, CH₂), 20.14 (1C, CH₃), 18.80 (1C, CH₃).

HRMS [M+H+]: 166.1225
Isolated yield: 30%

### 2-((2,5-dimethylphenyl)amino)ethanol, starting from 2,5-dimethylaniline and ethylene glycol

¹H NMR (300 MHz, CDCl₃) δ: 6.96 (d, J=7.4 Hz, 1H), 6.53 (d, J=7.5 Hz, 1H), 6.49 (s, 1H), 3.87 (t, J=5.3 Hz, 2H), 3.35 (t, J=5.3 Hz, 2H), 2.31 (s, 3H), 2.14 (s, 3H).

¹³C NMR (300 MHz, CDCl₃) δ: 146.24 (1C, C), 137.15 (1C, C), 130.50 (1C, CH), 120.04 (1C, C), 118.59 (1C, CH), 111.51 (1C, CH), 61.68 (1C, CH₂), 46.48 (1C, CH₂), 20.93 (1C, CH₃), 17.45 (1C, CH₃).

HRMS [M+H+]: 166.1220
Isolated yield: 30%

### 2-(naphthalene-2-ylamino)ethanol, starting from 2-amino naphthalene and ethylene glycol

¹H NMR (300 MHz, CDCl₃) δ: 7.68 (dd, J=8.1, 0.6 Hz, 1H), 7.64 (d, J=8.6 Hz, 1C), 7.62 (dd, J=8.1;0.6 Hz, 1H), 7.37 (ddd, J=8.2;6.8;1.3 Hz, 1C), 7.22 (ddd, J=8.1;6.9;1.2 Hz, 1C), 6.94 (dd, J=8.7;2.4 Hz, 1C), 6.90 (d, J=2.3 Hz, 1C), 3.91 (t, J=5.2 Hz, 2H), 3.42 (d, J=5.2 Hz, 2H), 3.00 (s, 1H).

¹³C NMR (300 MHz, CDCl₃) δ: 145.5 (1C, C), 135.16 (1C, C), 129.22 (1C, CH), 128.05 (1C, C), 127.79 (1C, CH), 126.56 (1C, CH), 126.17 (1C, CH), 122.53 (1C, CH), 118.38 (1C, CH), 105.64 (1C, CH), 61.20 (1C, CH₂), 46.57 (1C, CH₂).

HRMS [M+H+]: 188.1058
Isolated yield: 19%

### 2-((3-fluorophenyl)amino)ethanol, starting from 3-fluoroaniline and ethylene glycol

¹H NMR (300 MHz, CDCl₃) δ: 7.03 (td, J=8.1;6.7 Hz, 1C), 6.34 (m, 2H), 6.26 (dt, J=11.5;2.3 Hz, 1C), 3.76 (t, J=5.2 Hz, 2H), 3.21 (t, J=5.2 Hz, 2H).

¹³C NMR (300 MHz, CDCl₃) δ: 162.48 (1C, C), 149.78 (1C, C), 130.40 (1C, CH), 109.07 (1C, CH), 104.39 (1C, CH), 99.96 (1C, CH), 61.07 (1C, CH₂), 45.94 (1C, CH₂).

HRMS [M+H+]: 156.0807
Isolated yield: 32%

### 2-((2-fluorophenyl)amino)ethanol, starting from 2-fluoroaniline and ethylene glycol

¹H NMR (300 MHz, CDCl₃) δ: 7.00 (m, 2H), 6.76 (td, J=8.4;1.5 Hz, 1H), 6.66 (m, 1H), 3.86 (t, J=5.2 Hz, 2H), 3.35 (t, J=5.2 Hz, 2H).

¹³C NMR (300 MHz, CDCl₃) δ: X (1C, C), X (1C, C), 124.76 (1C, CH), 117.58 (1C, CH), 114.88 (1C, CH), 112.74 (1C, CH), 61.38 (1C, CH₂), 46.04 (1H, CH₂).

HRMS [M+H+]: 156.0811
Isolated yield: 23%

### 2-((2-(trifluoromethyl)phenyl)amino)ethanol, starting from 2-trifluoromethylaniline and ethylene glycol

¹H NMR (300 MHz, CDCl₃) δ: 7.81 (ddd, J=8.0;1.6;0.5 Hz, 1H), 7.21 (m, 1H), 6.58 (m, 2H), 4.34 (t, J=4.6 Hz, 2H), 3.87 (t, J=4.6 Hz, 2H).

¹³C NMR (300 MHz, CDCl₃) δ: 168.78 (1C, C), 150.97 (1C, C), 134.79 (1C, CH), 131.63 (1C, CH), 117.19 (1C, CH), 116.76 (1C, CH), 66.50 (1C, CH₂), 61.96 (1C, CH₂). Isolated yield: 41%

### 2-((2-(trifluoromethoxy)phenyl)amino) ethanol, starting from 2-methoxyaniline and ethylene glycol

¹H NMR (300 MHz, CDCl₃) δ: 7.07 (m, 2H), 6.70 (dd, J=8.5;1.5 Hz, 1H), 6.62 (m, 1 H), 3.78 (t, J=5.2 Hz, 2H), 3.28 (t, J = 5.2 Hz, 2H).

¹³C NMR (300 MHz, CDCl₃) δ: 140.88 (1C, C), 134.89 (1C, C), 128.08 (1C, CH), 121.39 (1C, CH), 117.40 (1C, CH), 112.75 (1C, CH), 61.49 (1C, CH₂), 46.05 (1C, CH₂). Isolated yield: 8%

### 2-((3,4,5-(trimethoxy)phenyl)amino)ethanol, starting from 3,4,5-trimethoxyaniline and ethylene glycol

¹H NMR (400 MHz, CDCl₃) δ: 5.89 (s, 2H), 3.83 (t, J=5.3 Hz, 2H), 3.81 (s, 6H), 3.76 (s, 3H), 3.27 (t, J = 5.3 Hz, 2H).

¹³C NMR (100 MHz, CDCl₃) δ: 153.9 (1C, C), 144. (1C, C) 9, 92.6 (1C, CH), 90.8 (1C, CH), 61.3, (1C, CH₃) 61.1 (1C, CH₃) 55.9, (1C, CH₂) 46.6 (1C, CH₂).

HRMS [M+H+]: 228.1230
Isolated yield: 16%

### Bibliographic references:

1. "Borrowing Hydrogen in the Activation of Alcohols". Advanced Synthesis Catalysis, Volume 349, Issue 10, pp. 1555-1575. Jul. 2, 2007.
2. "Catalytic carbonyl addition through transfer hydrogenation: a departure from preformed organometallic reagents". ACIEE (Angewandte Chemical International Edition in English), Vol. 48(1), 34-46 (Online ISSN: 1521-3773). 2009.
3. "Hydrogen Autotransfer in the N-Alkylation of Amines and Related Compounds using Alcohols and Amines as Electrophiles". Chemicals Review, 110 (3), pp 1611-1641. 2010.
4. "Substitution of alcohols by N-nucleophiles via transition metal-catalyzed dehydrogenation". Chemical Society Review, 44, pp. 2305-2329. 2015.
5. "Heterogeneous catalysis for the direct synthesis of chemicals by borrowing hydrogen methodology". Catalysis Science & Technology, 5, 1412-1427. 2015.
6. "Recent Developments in Methodology for the Direct Oxyamination of Olefins". Chemistry A European Journal. Vol. 17, Issue 1, pp. 58-76. Jan. 3, 2011.
7. "A sustainable catalytic pyrrole synthesis". Nature Chemistry 5, 140-144. Jan. 20, 2013.
8. "An Efficient Synthesis of Organic Carbonates using Nanocrystalline Magnesium Oxide". Advanced Synthesis & Catalysis, Vol. 349, Issue 10, pp 1671-1675. 2007.
9. "Tandem Synthesis of β-Amino Alcohols from Aniline, Dialkyl Carbonate, and Ethylene Glycol". Industrial & Engineering Chemistry Research, 47 (8), pp 2484-2494. 2008.
10. "A Bifunctional Pd/MgO Solid Catalyst for the One-Pot Selective N-Monoalkylation of Amines with Alcohols". Chemistry A European Journal, Vol. 16, Issue 1, pp. 254-260. 2010.

## Claims

1. Method for obtaining β-amino alcohols which, starting from an amine and a diol/water mixture, **is characterized by** being carried out in a heterogeneous catalytic system and comprise the following steps:
- a solid catalytic mixture comprising a supported metal and a solid inorganic coactivator with acid-base character is added to the starting amine,
- then, the diol/water mixture is added,
- subsequently, the reaction is carried out at a temperature of between 130 and 200°C, during 2-24 hours,
- the reaction product, comprising β-amino alcohol, is filtered, through a microporous filter, by means of at least a first extraction with hexane and, subsequently, at least a second extraction with ethyl acetate to purify the obtained β-amino alcohol.

2. Method according to claim 1, **characterized in that** the amine is of the H₂N-Ar type, wherein Ar is one of the following elements: phenyl, ortho/meta/para methylphenyl, ortho/meta/para nitrophenyl ortho/meta/para fluorophenyl, ortho/meta/para aminophenyl, ortho/meta/para trifluoromethylphenyl, ortho/meta/para trifluoromethoxyphenyl, 2,3; 3,4; 4,5; 2.4; 2,5-dimethylphenyl, 3,4,5-trimethylphenyl, anthracyl or naphthyl.

3. Method according to claim 1, **characterized in that** the amine is a heterocyclic amine.

4. Method according to claim 1, **characterized in that** after obtaining the β-amino alcohols, the catalytic mixture is recovered through a filtrate and washed with hexane/ethyl acetate and, subsequently, dried between 140 and 160°C, for 2 to 6 hours.

5. Method according to claim 1, **characterized in that** the diol of the diol/water mixture is selected from the following elements: 1,2-ethanediol (ethylene glycol), 1,2-propanediol, 2,3-butanediol, 1-phenylethane-1,2-diol, 1,3-propanol, 1,2-cycloexanediol, 1,3-butanediol, di-ethylene glycol, tri-ethylene glycol, tetraethylene glycol and the glycerol triol, and cyclic derivatives of them.

6. Method according to claim 1, **characterized in that** the inorganic solid coactivator with acid-base character is selected from the following elements: CaO, BaO, ZnO, CeO₂, Al₂O₃, MgO, TiO₂, SiO₂.

7. Method according to claim 1, **characterized in that** the supported metal is a metal selected from the following elements: transition metals of groups IIIB to IIB of the periodic system of elements, oxides and hydroxides thereof and a Pt-Sn alloy.

8. Method according to claim 7, **characterized in that** the supported metal is a metal selected from the following elements: Pd, Au, Pt, Cu, Ag, Ru, oxides and hydroxides thereof.

9. Method according to claim 1, **characterized in that** the metal is supported on a solid substrate that is selected from the following elements: TiO₂, Al₂O₃, SiO₂, FeO₃, Fe₂O₃, MgO, GaO₂, CeO₂, NiO, zeolites, graphite, graphene, graphene oxide, diamond, carbon nanotubes, carbon black.

10. Method according to claim 1, **characterized in that** the catalytic mixture comprises palladium supported on carbon and ZnO.
